# EUROPEAN PATENT APPLICATION

(11) **EP 3 895 624 A1**
(43) Date of publication of application: **20.10.2021**
(21) Application number: 20170089.5
(22) Date of filing: 17.04.2020
(51) Int. Cl.: A61B 17/04, A61F 2/08

(54) **MEDICAL IMPLANT, MEDICAL DEVICE, SEPARATION DEVICE, FELTING DEVICE AND METHODS**

(71) Applicant: ZuriMED Technologies AG, 8008 Zürich (CH)
(72) Inventor: Bachmann, Elias, 8006 Zürich (CH); Li, Xiang, 8126 Zumikon (CH)
(74) Representative: Schulz Junghans Patentanwälte PartGmbB

(57) **Abstract**

The invention relates to a medical implant (100) extending between a first end (103) and a second end (104), wherein the medical implant (100) comprises or consists of a plurality of fibers (101), the medical implant (100) comprising a main section (105) and a first connecting section (110) configured to be connected to soft biological tissue (500) or to a medical textile (300), particularly comprising or consisting of a felt material, wherein the first connecting section (110) extends towards the first end (103), and wherein the fibers (101) are connected in the main section (105) and separated from each other in the first connecting section (110).

Furthermore, the invention relates to a medical device (600) comprising the medical implant (100), a felting device (700) and a separation device (800), methods for producing a medical implant (100) and a medical device (600), and a method for biological soft tissue repair.

## Description

The invention relates to a medical implant, a medical device, a separation device and a method for producing a medical implant, a felting device and a method for producing a medical device and a method for soft tissue repair using the medical implant and/or the medical device. The implant and device are suitable, in particular, for orthopedic applications such as tendon and ligament repair.

Surgical suture is a medical device used to hold body tissues together after an injury or surgery whereas surgical knots are used to secure the sutures.

Braided or twisted sutures and suture-tapes are used in surgical indications, where tissue needs to be closed, anchored, repaired, re-connected or reconstructed. For load bearing indications, in which the suture needs to hold the tissue in place without elongating or rupturing, high strength sutures are used which are normally a braiding of many small fibers.

In addition, flat, two-dimensional structures such as patches are commonly used as medical devices in surgical procedures to optimize stress distribution of sutured tissue. Such devices have the benefit of distributing pressure over a larger area, however lack of strong tensile properties due to their structure.

A new technique for tightly connecting soft tissue to felt patches by advancing a felting needle comprising barbs through the tissue and the felt patch to distribute single fibers of the felt patch into the tissue, has recently been introduced.

However, for many surgical applications involving soft tissue repair (e.g. the orthopedic applications mentioned above) medical devices combining the high tensile strength of medical implants with the pressure-distributing abilities of patches would be highly desirable. For orthopedic soft tissue fixation applications, this would also minimize the risk of implant cut through, loosening, knot slippage or opening.

Therefore, the objective underlying the present invention is to provide a medical device combining high tensile strength with the ability to distribute pressure over an area.

This objective is attained by the subject matter of the independent claims. Embodiments of the invention are specified in the dependent claims and described hereafter.

A first aspect of the invention relates to a medical implant extending between a first end and a second end, wherein the medical implant comprises or consists of a plurality of fibers, wherein the medical implant comprises a main section and a first connecting section configured to be connected to soft biological tissue or to a medical textile, particularly a medical textile comprising or consisting of a felt material, wherein the first connecting section extends towards the first end, and wherein the fibers are connected, particularly braided, twisted, knitted or felted, in the main section and wherein the fibers are separated from each other in the first connecting section.

In particular, the medical implant is a medical textile that is braided, knitted, or twisted, comprising or consisting feltable fiber on the first end and the second end. Particularly, the medical implant is a suture, a tape or a mesh.

The separated fibers are configured to be connected to a medical textile or to tissue by positioning the first connecting section on the medical textile and repeatedly advancing a needle comprising at least one barb through the first connecting section through the medical textile. This technique is also designated herein as "felting" or "needle felting". Felting of synthetic fibers is known from various fields and has recently been introduced as a surgical technique.

By means of the separated fibers of the first connecting section, which are spread over a larger area than in the main section of the medical implant, pressure can be advantageously distributed over a larger area when the connecting section is connected to soft tissue (directly or via a medical textile, e.g. from a felt material), thereby improving the stability of the mechanical connection. At the same time, the good tensile strength of the medical implant is retained due to the braided or twisted main section.

The medical implant may be used as a medical device on its own (i.e. by connecting the first connecting section to soft tissue directly) or may constitute a medical device combined with a medical textile, particularly a medical textile comprising or consisting of a felt material.

According to the first aspect, the first connecting section extends towards the first end. In other words, the first connecting section extends between the main section and the first end.

The fibers are separated from each other in the first connecting section. That means that, as opposed to the main section, the fibers in the first connecting section are not connected, particularly braided (i.e. entangled), twisted, knitted or felted, with neighboring fibers. Of course, the fibers may still contact neighboring fibers in the first connecting section.

In certain embodiments, the medical implant comprises or consists of absorbable fibers, particularly polyglycolic acid, polylactic acid, polydioxanone, or caprolactone. Therein, the term absorbable relates to materials which are degraded after implantation in a human or animal body.

In certain embodiments, the medical implant comprises or consists of non-absorbable fibers, particularly silk, polypropylene, polyester or polyamide. Therein, the term non-absorbable relates to materials which are not degraded after implantation in a human or animal body.

In certain embodiments, the medical implant comprises or consists of a combination of absorbable fibers, particularly polyglycolic acid, polylactic acid, polydioxanone, or caprolactone and non-absorbable fibers, particularly silk, polypropylene, polyester or polyamide.

In certain embodiments, the felt material (of the medical textile) comprises or consists of polyethylene (PE) or polytetrafluorethylene (PTFE).

In certain embodiments, the medical textile comprises or consists of decellularized collagen tissue, particularly from human or animal origin.

In certain embodiments, the medical implant comprises a second connecting section configured to be connected to soft biological tissue or to a medical textile, particularly comprising or consisting of a felt material, wherein the second connecting section extends towards the second end, wherein the fibers are separated from each other in the second connecting section.

The fibers are separated from each other in the second connecting section, i.e. the fibers in the second connecting section are not connected, particularly braided (i.e. entangled), twisted, knitted or felted, with neighboring fibers. Of course, the fibers may still contact neighboring fibers in the second connecting section.

A medical implant with connecting sections comprising separated fibers at each end can be used, e.g., as a standalone device (i.e. without an additional medical textile) by directly connecting soft tissue (by felting) to both ends of the implant. Alternatively, such a medical implant can be used in combination with a medical textile as a medical device according to this disclosure.

Further applications of the medical device include the repair or fixation of anatomical structures such as tendons, menisci, spinal disks or fascias, ligament constructions (collateral ligaments, cruciate ligaments, etc.), subcutaneous sutures, conventional suturing of skin closures, muscle, valves, and hollow organs, such as large vessels, bladder, esophagus, or intestine.

In certain embodiments, each of the separated fibers comprises a fiber end or a fiber loop positioned at the first end or the second end of the medical implant. In other words, the ends of the fibers can, but do not have to be positioned at the first end or the second end of the medical implant. Alternatively, the fibers may also form loops at the first end or the second end.

In certain embodiments, the main section and the first connecting section and/or second connecting section of the medical implant each comprise an area perpendicular to an extension direction of the medical implant, wherein the area of the first connecting section and/or second connecting section is greater than the area of the main section. In the special case of a circular cross-section of the main section and the connecting section, this difference in area will result in the connecting sections having a greater width compared to the main section. Accordingly, due to the lack of interconnections between the fibers, the individual fibers branch out or fan out in the first or second connecting section, resulting in a greater area compared to the main section. This is advantageous for connecting the connecting section to tissue or felt structures, since the area of the connecting section is increased, allowing a stronger connection by felting.

In certain embodiments, the first connecting section and/or the second connecting section extends over a length, wherein the length equals at least twice a width of the medical implant, particularly at least three times a width of the medical implant.

In certain embodiments, the first connecting section and/or the second connecting section forms a fan-like structure extending in a plane parallel to an extension direction of the medical implant.

In the context of the present specification, a "fan-like structure" is a planar (i.e. essentially two-dimensional) structure which branches out from the main section of the medical implant, resulting in a greater width of the respective connecting section in the direction of the plane, in which the fan-like structure extends.

Such a fan-like structure keeps its form and thus allows an especially easy handling. In addition, the spread-out fan-like structure allows an optimal force distribution and is easy to connect to medical textiles or soft tissue.

In certain embodiments, the first connecting section or the second connecting section forms a tubular structure, particularly comprising a circular or oval-shaped cross-sectional contour perpendicular to an extension direction of the medical implant.

In certain embodiments, an area or a width of the cross-sectional contour increases, particularly monotonously, towards the first end of the medical implant.

In certain embodiments, the cross-sectional contour is outwardly curved. Such a shape can also be described as a trumpet shape.

In certain embodiments, the fibers each comprise a plurality of barbs. These barbs improve interaction with a medical textile, particularly from a felt material, or with biological soft tissue, when the first or second connecting section is connected to the medical textile or the tissue, thereby improving the mechanical connection.

In certain embodiments, the fibers of the medical implant comprise or consist of a biocompatible polymer, particularly polyethylene (PE, CAS No. 9002-88-4) polytetrafluorethylene (PTFE, CAS No. 9002-84-0), polyethylene terephthalate (PET, CAS No. 25038-59-9), poly(glycolide) (PGA, CAS No. 26124-68-5), poly(lactic-co-glycolic acid) (PLGA, CAS No. 26780-50-7), poly (L-lactic acid) (PLLA, CAS No. 33135-50-1), polycaprolactone (PCL, CAS No. 24980-41-4), 1,2-propanediol (PDO, CAS 57-55-6) or 1,3-propanediol (PDO, CAS No. 504-63-2).

In certain embodiments, the medical implant, particularly the suture, comprises a diameter of 0,05 mm to 3 mm, particularly 0,2 mm to 2 mm, more particularly 0,5 mm to 1 mm.

A second aspect of the invention relates to a medical device comprising at least one medical implant according to the first aspect of the invention.

In certain embodiments, the medical device further comprises a medical textile, particularly comprising or consisting of a felt material, wherein the separated fibers of the first connecting section and/or the second connecting section of the medical implant extend into or through the medical textile, particularly into or through the felt material of the medical textile, to connect the medical implant to the medical textile.

Advantageously, this allows a mechanically strong connection between an implant and a medical textile in an easy and cost-efficient manner.

In certain embodiments, the medical implant, particularly the suture, comprises 20 to 20000 fibers, particularly 200 to 10000 fibers, more particularly 1000 to 6000 fibers.

In certain embodiments, the medical textile comprises a density of 0.1 g/cm³ to 0,5 g/cm³ in a dry state. In certain embodiments, the medical textile comprises a density of 0.3 g/cm³ to 0,8 g/cm³ in a wet state.

In certain embodiments, the medical implant comprises a tear strength of 10 N to 3000 N, particularly 40 N to 800 N. In certain embodiments, the medical implant comprises a tear strength of at least 200 N.

In certain embodiments, the medical textile comprises a tear strength of 10 N to 3000 N, particularly 40 N to 800 N. In certain embodiments, the medical textile comprises a tear strength of at least 200 N.

In certain embodiments, the medical device comprises a tear strength of 10 N to 3000 N, particularly 40 N to 800 N. In certain embodiments, the medical device comprises a tear strength of at least 200 N. The term "tear strength" when relating to the medical device designates the force at which the medical device tears (by tearing of the medical implant, particularly the suture, tearing of the medical textile or tearing of the connection between the medical implant and the medical textile, when the medical implant (or medical implants in case of more than one medical implant) and the medical textile are pulled apart.

In certain embodiments, the medical device comprises a stiffness of 30 N/mm to 300 N/mm, particularly 60 N/mm to 250 N/mm, more particularly 130 N/mm to 220 N/mm. Therein the term "stiffness" when relating to the medical device designates a force required per unit of length of elongation of the medical device when the medical textile and the medical implant, particularly the suture, (or medical implants in case of more than one medical implant) are pulled apart.

In certain embodiments, the medical textile comprises a first portion and at least one second portion, wherein the second portion comprises a higher stiffness than the first portion. In other words, the medical textile comprises at least one reinforced area (second portion) of higher stiffness. The second (reinforced) portion may be obtained by (pre-) felting, that is advancing a felting needle comprising at least one barb through the medical textile at certain areas resulting in an increased entanglement of the fibers of the medical textile and thereby a higher stiffness.

This allows non-uniform stiffness properties over the medical textile and can be advantageous in applications where different lateral or bending stiffness are required.

In certain embodiments, the medical textile extends along a plane, wherein particularly the medical textile comprises a flat shape.

In the context of the present specification, the term "flat shape" particularly refers to a medical textile having a length, a width and a thickness perpendicular to the length and the width, wherein a ratio between the length and the thickness and/or a ratio between the width and the thickness is at least 2:1, particularly at least 5:1, more particularly at least 10:1, even more particularly at least 20:1.

In certain embodiments, the medical textile comprises a thickness, particularly perpendicular to the plane, of 0,1 mm to 5 mm, particularly 0,5 mm to 3 mm, more particularly 1 mm to 2 mm.

In certain embodiments, the medical device comprises a first medical implant according to the first aspect of the invention, wherein the first connecting section and/or the second connecting section of the first medical implant forms a fan-like structure extending in a plane parallel to an extension direction of the first medical implant, and wherein the first medical implant is connected to the planar medical textile, such that the first medical implant extends from the medical textile parallel to the plane of the medical textile.

By the fan-like structure, the implant is pre-oriented parallel to the patch, such that it is easy to establish a robust connection in this orientation with optimal force distribution.

In certain embodiments, the medical device comprises a second medical implant according to the first aspect of the invention, wherein the first connecting section or the second connecting section of the second medical implant forms a tubular structure, particularly comprising a circular or oval-shaped cross-sectional contour perpendicular to an extension direction of the medical implant, wherein particularly an area or a width of the cross-sectional contour increases, particularly monotonously, towards the first end of the second medical suture, wherein the second medical implant is connected to the planar medical textile, such that the second medical implant extends from the medical textile perpendicular to the plane of the medical textile.

By the tubular structure, the implant is pre-oriented perpendicular to the medical textile, such that it is easy to establish a robust connection in this orientation with optimal force distribution.

In certain embodiments, the medical textile extends along a curved surface, wherein particularly the medical textile comprises a flat shape. In certain embodiments, the medical textile comprises a tubular shape, wherein the medical textile comprises an opening configured for insertion of a soft biological tissue, particularly a tendon, more particularly an end of a tendon, into the medical textile, wherein particularly the first connecting section or the second connecting section of the medical implant forms a tubular structure, more particularly comprising a circular or oval-shaped cross-sectional contour perpendicular to an extension direction of the medical implant. In particular, the tubular structure is arranged in the tubular medical textile contacting the inner circumference of the medical textile or the tubular structure is arranged at least partially around the outer circumference of the medical textile. In particular, the separated fibers of the tubular structure extend into or through the medical textile, particularly into or through the felt material of the medical textile, to connect the medical implant to the medical textile.

This embodiment allows an easy and especially tight connection of a tendon end to an implant in the axial direction, and optimized force distribution is this direction.

In certain embodiments, the medical device comprises a first medical textile, particularly comprising or consisting of a felt material, and a second medical textile, particularly comprising or consisting of a felt material, wherein the first connecting section and/or the second connecting section of the medical implant is arranged between the first medical textile and the second medical textile, and wherein the separated fibers of the first connecting section and/or the second connecting section of the medical implant extend into or through the first medical textile or the second medical textile, particularly into or through the felt material of the first medical textile and the second medical textile.

This sandwich-like arrangement maximizes the interweaving of the fibers and thus further improves the stability of the connection to the implant, and results in an advantageous force distribution over the two medical textiles.

In certain embodiments, the medical device further comprises at least one anchoring element configured to be inserted into a bone, wherein particularly the anchoring element is connected to the medical implant.

By such an anchoring element, the medical device may be fixed to a bone, which is necessary e.g. for rotator cuff repair.

In the context of the present specification, the term anchoring element refers to any member which is configured to be fixed inside a bone, e.g. an anchor, a peg, a screw, a nail or a bolt.

In certain embodiments, the medical device further comprises a further fixing or anchoring part, particularly a screw, a washer, a pin or a plate.

A third aspect of the invention relates to a separation device for producing a medical implant according to the first aspect of the invention, comprising a first roller rotatably mounted on a first shaft extending along a longitudinal axis and a second roller, wherein the first roller comprises a plurality of spikes extending radially with respect to the longitudinal axis, and wherein the second roller comprises a plurality of grooves extending in a circumferential direction around the second roller, wherein each groove is aligned with a corresponding spike of the first roller, such that the spikes of the first roller are able to insert into and move through the respective grooves when the first roller is rotated around the first shaft, and wherein the first roller and the second roller are separated by a gap configured to receive a medical implant comprising a plurality of braided, twisted, knitted or felted fibers, and wherein the device is configured to separate the fibers of the medical implant by means of the spikes when the first roller is rotated around the first shaft, thereby generating the first connecting section or the second connecting section of the medical implant.

This device facilitates production of medical implants according to the invention in a fast, cost-efficient and standardized manner, particularly automatically.

In certain embodiments, the second roller is rotatably mounted on a second shaft parallel to the first shaft. In particular, the second shaft is driven to actively rotate the second roller, more particularly in an opposite direction in respect of the first roller. In this manner, the first or second end of the medical implant may be moved in the gap to elongate the first or second connecting section where the fibers are separated. Alternatively, the second roller may be passively rotatable (not driven), or the second roller may be mounted in a fixed manner (non-rotatable).

In certain embodiments, the device comprises a transport mechanism configured to transport the medical implant, particularly the first end or the second end of the medical implant, towards the gap between the first roller and the second roller.

In certain embodiments, the transport mechanism comprises a third roller and a fourth roller, wherein the third roller is rotatably mounted on a third shaft and the fourth roller is rotatably mounted on a fourth shaft, wherein the fourth shaft is parallel to the third shaft, and wherein a gap for receiving a medical implant is formed between the third roller and the fourth roller, and wherein the transport mechanism is configured to move the medical implant by rotating the third roller and the fourth roller in opposite directions. The third roller and the fourth roller constitute a conveyer mechanism to move the medical implant towards the first and second roller.

In certain embodiments, the transport mechanism comprises a fifth roller and a sixth roller, wherein the fifth roller is rotatably mounted on a fifth shaft and the sixth roller is rotatably mounted on a sixth shaft, wherein the fifth shaft is parallel to the sixth shaft, and wherein a gap for receiving a medical implant is formed between the fourth roller and the fifth roller, and wherein the transport mechanism is configured to move the medical implant by rotating the fifth roller and the sixth roller in opposite directions. The fifth roller and the sixth roller constitute a conveyer mechanism to move the medical implant towards the first and second roller.

A fourth aspect of the invention relates to a method for producing a medical implant according to the first aspect of the invention using a device according to the third aspect of the invention, wherein the method comprises providing a medical implant comprising a plurality of braided, twisted, knitted or felted fibers in the gap between the first roller and the second roller, and rotating the first roller around the first shaft, such that the fibers of the medical implant are separated by the spikes, thereby generating the first connecting section or the second connecting section of the medical implant.

A fifth aspect of the invention relates to a felting device for producing a medical device according to the second aspect of the invention, wherein the device comprises a surface for arranging a medical textile, particularly comprising or consisting of a felt material, and at least one needle comprising at least one barb, wherein the device is configured to repeatedly advance the needle through the medical textile arranged on the surface and through the first connecting section or the second connecting section of a medical implant according to the first aspect of the invention which is arranged on the medical textile, such that the separated fibers of the first connecting section or the second connecting section of the medical implant are advanced into or through the medical textile, particularly into or through the felt material of the medical textile, to connect the medical implant to the medical textile.

Such a device is able to quickly and easily establish a very robust mechanical connection between a medical textile and an implant.

In certain embodiments, the surface is arranged in a slot, particularly between two side walls arranged opposite each other along a first direction. More particularly, the slot is open along a second direction perpendicular to the first direction. For instance, the slot may be part of a casing or box or the like.

In certain embodiments, the device comprises a bottom part comprising the slot and a top part comprising the at least one needle.

In certain embodiments, the device, particularly the top part, comprises a plurality of needles arranged parallel to each other in a two-dimensional array.

In certain embodiments, the device comprises a drive mechanism for moving the needles to repeatedly advance at least a subset of the needles through the medical textile arranged on the surface and through a first connecting section or the second connecting section of a medical implant according to the first aspect of the invention which is arranged on the medical textile.

In certain embodiments, the surface comprises at least one hole, wherein the at least one hole is aligned with the at least one needle, such that the at least one needle is insertable into the at least one hole when the at least one needle is repeatedly advanced through the medical textile arranged on the surface and through the first connecting section or second connecting section of the medical implant which is arranged on the medical textile.

In certain embodiments, the surface comprises a plurality of holes, wherein the holes are aligned with the plurality of needles, such that each needle is insertable into a corresponding hole when the needles are repeatedly advanced through the medical textile arranged on the surface and through the first connecting section or second connecting section of the medical implant which is arranged on the medical textile.

These embodiments have the advantage that the at least one needle can be advanced deeper into the medical textile and the connecting section to achieve a more thorough connection between the medical implant and the medical textile or to achieve the connection faster without hitting the surface which may damage the needle.

Alternatively, the surface may consist of a material allowing penetration of the at least one needle into the surface without damaging the needle (e.g. a soft, flexible material such as rubber or a foam material).

In certain embodiments, the felting device comprises a transport mechanism for providing the medical textile in the slot, particularly below the medical implant, such that the medical textile is arranged on the surface.

In certain embodiments, the felting device comprises a cutting device for cutting the medical textile into a desired shape.

A sixth aspect of the invention relates to a system comprising a separation device according to the third aspect of the invention and a felting device according to the fifth aspect of the device.

In certain embodiments, the system comprises a transport mechanism for moving a medical implant, particularly the first connecting section or the second connecting section of the medical implant, from the separation device to the felting device.

In certain embodiments, the transport mechanism is configured to move the first connecting section or the second connecting section of the medical implant from the separation device, particularly from the gap between the first roller and the second roller, onto a medical textile, particularly comprising or consisting of a felt material, the medical textile being arranged on the surface of the felting device.

In certain embodiments, the transport mechanism comprises a third roller and a fourth roller, wherein the third roller is rotatably mounted on a third shaft and the fourth roller is rotatably mounted on a fourth shaft, wherein the fourth shaft is parallel to the third shaft, and wherein a gap for receiving a medical implant is formed between the third roller and the fourth roller, and wherein the transport mechanism is configured to move the medical implant by rotating the third roller and the fourth roller in opposite directions.

In certain embodiments, the transport mechanism comprises a fifth roller and a sixth roller, wherein the fifth roller is rotatably mounted on a fifth shaft and the sixth roller is rotatably mounted on a sixth shaft, wherein the fifth shaft is parallel to the sixth shaft, and wherein a gap for receiving a medical implant is formed between the fourth roller and the fifth roller, and wherein the transport mechanism is configured to move the medical implant by rotating the fifth roller and the sixth roller in opposite directions.

A seventh aspect of the invention relates to a method for producing a medical device according to the second aspect of the invention, particularly using a device according to the fifth aspect of the invention, wherein a medical textile, particularly comprising or consisting of a felt material, is provided, and wherein the first connecting section or the second connecting section of a medical implant according to the first aspect of the invention is arranged on the medical textile, and wherein at least one needle comprising at least one barb is repeatedly advanced through the medical textile and through the first connecting section or the second connecting section, such that the separated fibers of the first connecting section or the second connecting section of the medical implant are advanced into or through the medical textile, particularly into or through the felt material of the medical textile, to connect the medical implant to the medical textile.

In certain embodiments, a felting needle comprising at least one barb is advanced through the medical textile (also termed (pre)-felting) to generate at least one second portion, wherein the second portion comprises a higher stiffness than a first portion of the medical textile.

In certain embodiments, the medical textile is arranged on the surface of the device according to the fifth aspect of the invention.

In certain embodiments, the first connecting section or the second connecting section of the medical implant is arranged on a first medical textile, and a second medical textile is arranged on the first or second connecting section, and the at least one needle comprising the at least one barb is repeatedly advanced through the first medical textile, the first or second connecting section and the second medical textile.

In certain embodiments, the at least one needle is comprised in the device according to the fifth aspect of the invention.

In certain embodiments, the method further comprises, prior to arranging the first connecting section or the second connecting section on the medical textile, providing a medical implant comprising a plurality of braided, twisted, knitted or felted fibers in the gap between the first roller and the second roller of the separation device according to the third aspect, and rotating the first roller around the first shaft, such that the fibers of the medical implant are separated by the spikes, thereby generating the first connecting section or the second connecting section of the medical implant.

In certain embodiments, the method further comprises, prior to generating the first connecting section or the second connecting section of the medical implant, moving, particularly by means of the transport mechanism, the medical implant towards the separation device, particularly towards the gap between the first roller and the second roller, along a first direction, and, after generating the first connecting section or the second connecting section of the medical implant, moving, particularly by means of the transport mechanism, the medical implant from the separation device, particularly from the gap between the first roller and the second roller, towards the felting device, particularly towards the surface, along a second direction opposite the first direction.

A eighth aspect of the invention relates to a method for biological soft tissue repair, wherein the method comprises providing a medical implant according to the first aspect of the invention, and wherein the first connecting section or the second connecting section of the medical implant is arranged on a soft biological tissue, and wherein a needle comprising at least one barb is repeatedly advanced through the first connecting section or the second connecting section of the medical implant and the soft biological tissue to connect the medical implant to the soft biological tissue, wherein particularly the method is performed outside of a human or animal body.

In certain embodiments, the soft biological tissue is a tendon, particularly a rotator cuff tendon or a biceps tendon, or a ligament, particularly an anterior cruciate ligament.

In certain embodiments, the soft biological tissue is muscle tissue, particularly comprising at least two separated muscle portions, wherein the medical implant is used to close the separated muscle portions.

An ninth aspect of the invention relates to a method for biological soft tissue repair, wherein the method comprises providing a medical device according to the second aspect of the invention, and wherein the medical textile of the medical device is arranged on a soft biological tissue, and wherein a needle comprising at least one barb is repeatedly advanced through medical textile, particularly the felt material of the medical textile, and the soft biological tissue to connect the medical device to the soft biological tissue, wherein particularly the method is performed outside of a human or animal body.

In certain embodiments, the soft biological tissue is a tendon or a ligament.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.
- Fig. 1: schematically depicts a method of production of a medical device according to the invention;
- Fig. 2: shows a first embodiment of a medical device according to the invention;
- Fig. 3: shows a second embodiment of a medical device according to the invention;
- Fig. 4: shows a third embodiment of a medical device according to the invention;
- Fig. 5: shows further embodiments of a medical device according to the invention;
- Fig. 6: schematically depicts surgical applications of the medical device according to the invention;
- Fig. 7: schematically depicts a system comprising a felting device and a separation device according to the invention.

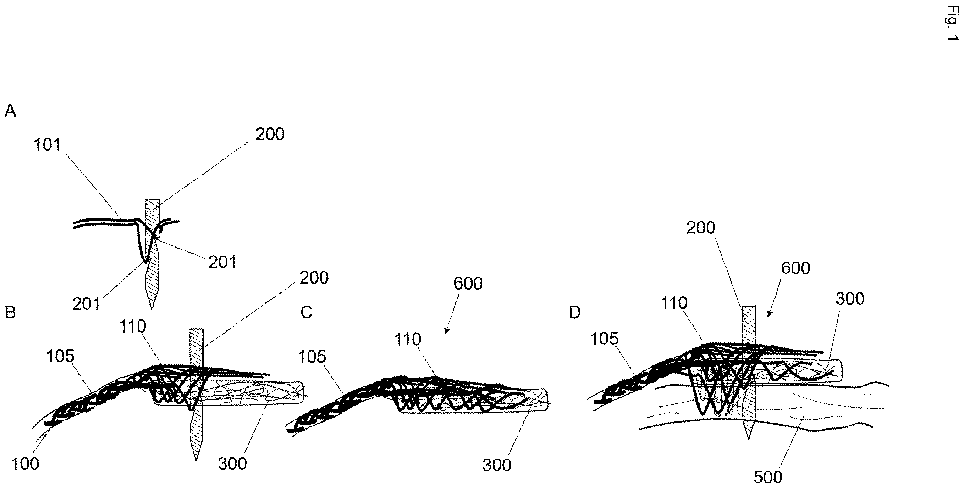

Fig. 1 is a schematic representation of the manufacture and use of a medical device 600 according to the invention. Fig. 1A shows a felting needle 200 comprising a plurality of barbs 201 and fibers 101 of the braided medical implant 100 in the form of a braided medical suture entangled with the barbs 201 of the needle 200. For simplicity only two fibers 101 are drawn, although the person skilled in the art will readily appreciate that in an actual felting procedure many more fibers 101 will be entangled with the barbs 201.

Fig. 1B depicts a medical implant 100 that is a braided medical suture comprising a first connecting section 110 adjacent to a main section 105. The medical implant 100 is connected to a medical textile 300 in form of a patch from a felt material by repeatedly advancing the needle 200 through the first connecting section 110 and the felt material of the medical textile 300.

Fig. 1C shows the results of the procedure illustrated in Fig. 1B: a medical device 600 comprising the medical implant 100 and the medical textile 300 from a felt material, wherein the fibers 101 of the connecting section 110 of the medical implant 100 have been intricately connected to the medical textile 300 by the felting method described above.

Furthermore, Fig. 1D shows a medical application of the medical device 600 inside or outside of the human or animal body. Therein, the medical textile 300 is connected to biological soft tissue 500 (e.g. tendon or muscle), by repeatedly advancing a surgical felting needle 200, similar to the one shown in Fig. 1A, through the medical textile 300 and the soft tissue 500. By the felting needle 200, individual fibers of the medical textile 300 and of the first connecting section 110 of the medical implant 100 are disposed in the biological soft tissue 500, thereby connecting the medical device 600 to the soft tissue 500.

In summary, Fig. 1 shows how two components (braided implant 100 and medical textile 300 in form of a felt patch) are combined using a felting technique to obtain a strong medical device 600 with a high tensile load due to the braided implant and the ability to be connected to soft tissue 500 in an easy manner with optimal load distribution due to the medical textile 300. By a similar surgical felting technique, the medical device 600 is connected to soft tissue 500.

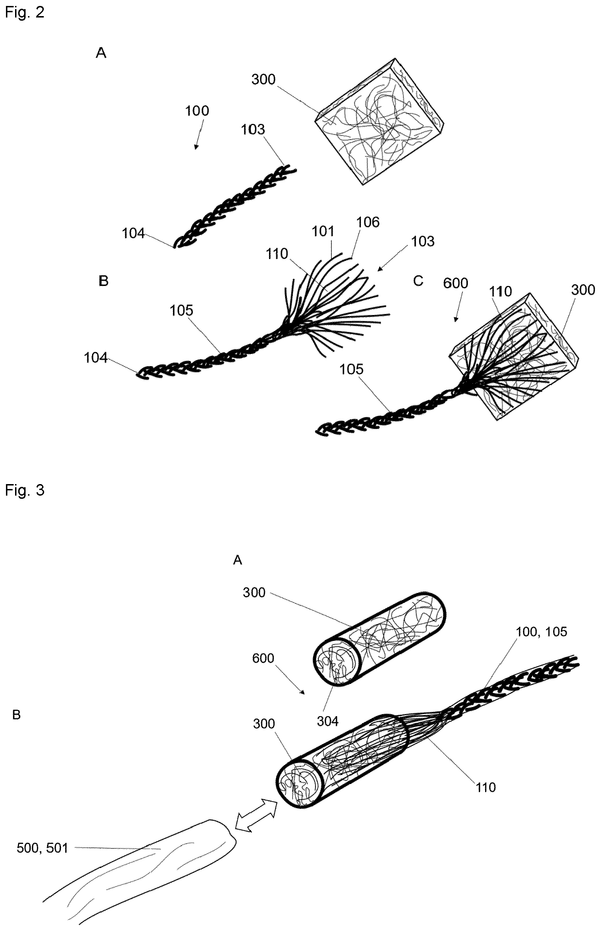

Fig. 2 shows a first embodiment of the medical implant 100 and medical device 600 according to the present invention comprising a braided medical implant 100 (medical suture) having a single first connecting section 110 forming a fan like structure combined with a planar rectangular medical textile 300, such as a felt patch.

Fig. 2A shows the initial components of the medical device 600, namely the braided medical implant 100 (medical suture) before the first connecting section 110 is formed, and the medical textile 300. The medical implant 100 extends along an extension direction from the first end 103 towards the second end 104. By separating or disentangling the fibers 101 of the medical implant 100 at the first end 103, the first connecting section 110 forming a fan-like structure can be obtained (Fig. 2B). The fan-like structure extends in a plane and branches out from the main section 105 of the medical implant 100, resulting in a greater width of the first connecting section 110, compared to the main section 105.

As shown in Fig. 2C, the medical implant 100 shown in Fig. 2B can be combined with the medical textile 300 shown in Fig. 2A, by placing the first connecting section 110 on one surface of the medical textile 300 and connecting the first connecting section 110 to the medical textile 300 by repeatedly advancing a felting needle through the assembly, whereby the fibers 101 of the first connecting section 110 are entangled with the medical textile 300 forming a strong connection.

Fig. 3 illustrates a second embodiment of the medical device 600 according to the invention comprising a medical implant 100 (medical suture), with a tubular-shaped first connecting section 110 and a medical textile 300 in form of a hollow tube from a felt material. The fibers 101 of the first connecting section 110 are connected to the medical textile 300, preferably by inserting the first connecting section 110 into an opening 304 of the medical textile 300, and advancing a felting needle 200 repeatedly through the assembly to connect the fibers 101 with the felt material of the medical textile, thereby forming the medical device 600. Alternatively, it is also conceivable to arrange the first connecting section 110 around the outer surface of the medical textile 300 and connect the first connecting section 110 to the felt material by means of the felting needle 200. Subsequently, a biological soft tissue 500, preferably a tendon 501, can be inserted into the opening 304 of the medical textile 300 and connected to the medical device 600 by advancing a felting needle 200 through the medical textile 300 and the tendon 501.

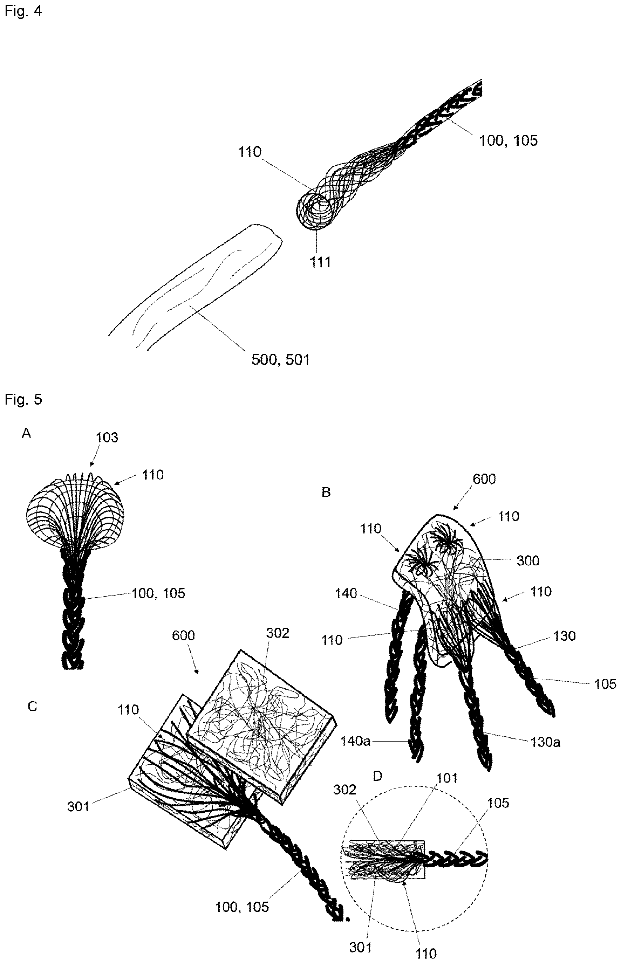

Fig. 4 depicts a further embodiment of the invention, where the medical implant 100 (medical suture) is used as a standalone medical device, i.e. without a medical textile 300. The first connecting section 110 of the medical implant 100 shown in Fig. 4 forms a hollow tubular or fingertrap-like structure comprising an opening 111 to insert and connect a soft biological tissue 500, such as a tendon 501 to the medical implant 100. An end of the tendon 501 is advanced into the opening 111 of the first connecting section 110 and mechanically connected by the felting technique, resulting in fibers 101 of the medical implant 100 being incorporated in the tendon 501 to generate a strong connection between the medical implant 100 and the tendon 501.

A number of further embodiments of the medical implant 100 and medical device 600 are shown in Fig. 5A-D. Fig. 5A depicts a medical implant 100 (medical suture) comprising a trumpet-like first connecting section 110, i.e. a first connecting section 110, with a circular cross-section perpendicular to the extension direction of the main section 105 of the medical implant 100, wherein the width of the first connecting section 110 increases monotonously towards the first end 103, and wherein the first connecting section 110 is outwardly curved towards the first and 103.

Fig. 5B illustrates a medical device 600 comprising a planar rectangular medical textile 300 in form of a felt patch combined with four medical implants 100 (sutures), namely a first medical implant 130, a second medical implant 140, a third medical implant 130a, and a fourth medical implant of 140a. The first medical implant 130 and the third medical implant 130a are formed as shown in Fig. 2B, i.e., with a fan-like first connecting section 110. To connect the first medical implant 130 and the third medical implant 130a to the medical textile 300, the first connecting sections 110 of the first and third medical implants 130, 130a are arranged on the upper surface of the medical textile 300 next to each other, such that the plane of the fan-like first connecting sections 110 is parallel to the plane of the medical textile 300, and connected by a felting needle 200. Accordingly, the first medical implant 130 and the third medical implant 130a extend from the medical textile 300 parallel to the plane of the medical textile 300.

In contrast, the second medical implant 140 and the fourth medical implant 140a are formed as shown in Fig. 5A, i.e., with a trumpet-shaped first connecting section 110, and have been connected to the medical textile 300 by placing the trumpet-shaped first connecting sections 110 onto the lower surface of the medical textile 300 in a manner such that the main sections 105 of the second and fourth medical implants 140, 140a extend from the medical textile 300 perpendicular to the plane the medical textile 300.

As is apparent from Fig. 5B, connections parallel to the plane of medical textile300 can be easily realized using the planar fan-like first connecting section 110 shown in Fig. 2 and connections perpendicular to the plane of the medical textile 300 can be easily obtained by using medical implants with a trumpet-like first connecting section 110 as shown in Fig. 5A.

Fig. 5C shows a further embodiment of the medical device 600 comprising a medical implant 100 (medical suture) with a fan-like first connecting section 110 (see Fig. 2B), as well as two rectangular planar medical textiles 301, 302 in the form of felt patches. To form the medical device 600, the first connecting section 110 of the medical implant 100 is first placed on the first medical textile 301, and optionally fixed to the first medical textile 301 by felting needle 200. Subsequently, the second medical textile 302 is placed on top of the connecting section 110 and attached to the connecting section 110 and the first medical textile 301 by advancing a felting needle repeatedly through the assembly. The result is a sandwich-like structure, where the medical implant 100 is placed between two medical textiles 301, 302, resulting in an extremely mechanically stable structure.

Fig. 5D is a cross-section of the medical device 600 shown in Fig. 5C in a plane perpendicular to the extension of the first medical textile 301 and the second medical textile 302. It is shown that the fibers 101 of the medical implant 100 are entangled into the felt material of the first medical textile 301 and the second medical textile 302.

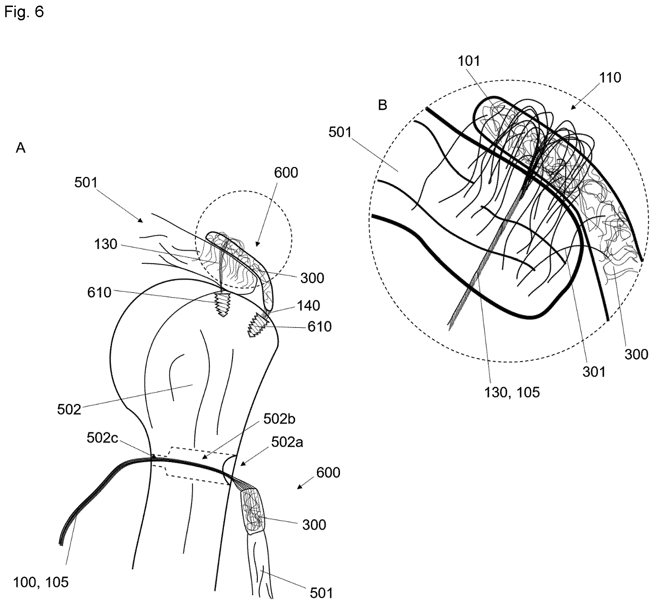

Fig. 6 shows examples of surgical applications of the medical implant 100 and medical device 600 according to the invention. Fig. 6 depicts a humerus bone 502, to which a rotator cuff tendon 501, e.g. supraspinatus tendon, is attached in a surgical rotator cuff repair procedure using a medical device 600 according to the invention.

Rotator cuff tears are one of the most frequent tendon injuries with reported failure rates between 30 and 70%. Especially early failure of repaired tendons and ligaments are caused by suture or knot failure where the suture pulling out of the tendon or through the bone. To ideally repair a soft-tissue, high initial fixation strength should be achieved to allow minimal gap formation and mechanical stability to allow a solid healing.

The medical device 600 comprises a first medical implant 130 and a second medical implant 140 (here, first and second medical sutures) connected by felting to a planar medical textile 300 from a felt material. The medical textile 300 has been tightly connected by felting to the tendon 501. Furthermore, the medical device 600 comprises additional anchoring elements 610 (e.g., bone anchors 610), which are attached to the first medical implant 130 and second medical implant 140, respectively.

A detailed view of the tendon 501 and medical device 600 is depicted in Fig. 6B. As shown in Fig. 6B, the first connecting section 110 of the first implant 130 has a trumpet-like shape similar to the one shown in Fig. 5A. The individual fibers 101 of the first connecting section 110 are shown protruding through the medical textile 300 and into the tendon 501. Furthermore, it is apparent from Fig. 6B that the main section 105 of the first medical implant 130 protrudes through the tendon 501.

In a typical method using the medical device 600 shown in Fig. 6A for rotator cuff repair, the medical device 600 is first prepared by connecting the first medical implant 130 with a trumpet shaped first connecting section 110 and the second medical implant 140 with a fan-like first connecting section 110 to the medical textile 300 by repeatedly advancing a felting needle 200 through the implants 130, 140 and the medical textile 300. This results in a similar configuration to the one shown in Fig. 5B, i.e. the first medical implant 130 is oriented perpendicular to the medical textile 300 and the second medical implant 140 is oriented parallel to the medical textile 300. Next, the first medical implant 130 is advanced through the tendon 501, e.g. by means of a surgical needle. Subsequently, a surgical felting needle is repeatedly advanced through the assembly formed by the tendon 501, the medical textile 300 and the first medical implant 130 to achieve a tight connection due to fibers 303 of the medical textile 300 and fibers 101 of the first connecting section 110 of the first medical implant 130 extending into the tendon 501. The anchoring elements 610 are then connected to the main section 105 of the first medical implant 130 and the second medical implant 140, respectively, and the construct comprising the tendon 501 and the medical device 600 is connected to the bone 502 by inserting the anchoring elements 610 into the bone 502 (e.g., by screwing). Finally, the first medical implant 130 and the second medical implant 140 are tightened to obtain the desired position of the tendon 501 on the bone 502 and tightly connect the tendon 501 to the bone 502.

The lower portion of Fig. 6A shows a further possible use of the medical device 600 according to the invention, namely a biceps tendon repair after biceps tenodesis. Therein, the medical device 600 comprises a medical implant 100 (suture) with a tube-like connecting section 110 (see Fig. 3), a medical textile 300 in form of a hollow tube from a felt material and a biceps tendon 501. The first connecting section 110 of the medical implant 100 is connected to the medical textile 300 by felting as depicted in Fig. 3, and the biceps tendon 501 is inserted into an opening 304 (see Fig. 3) of the medical textile 300 and connected to the medical textile 300 by felting. The main section 105 of the medical implant 100 is then advanced through a bone tunnel 502a protruding through the bone 502. The bone tunnel 502a comprises a first section 502b and a second section 502c, wherein the first section 502c has a larger diameter compared to the second section 502c. Typically, the medical device 600 with the attached tendon 501 is advanced into the bone tunnel 502a by pulling on the end of the main section 105 of the medical implant 100, until a part of the tendon 501 is arranged in the first section 502b. Thereafter, the medical implant 100 is typically tightened to fix the medical device 600 in the bone tunnel 502a.

For simplicity, the medical devices 600 used in rotator cuff repair and biceps tendon repair are illustrated on the same bone 502 in Fig. 6A. However, typically, the two procedures will not be performed in combination on the same bone 502, but separately.

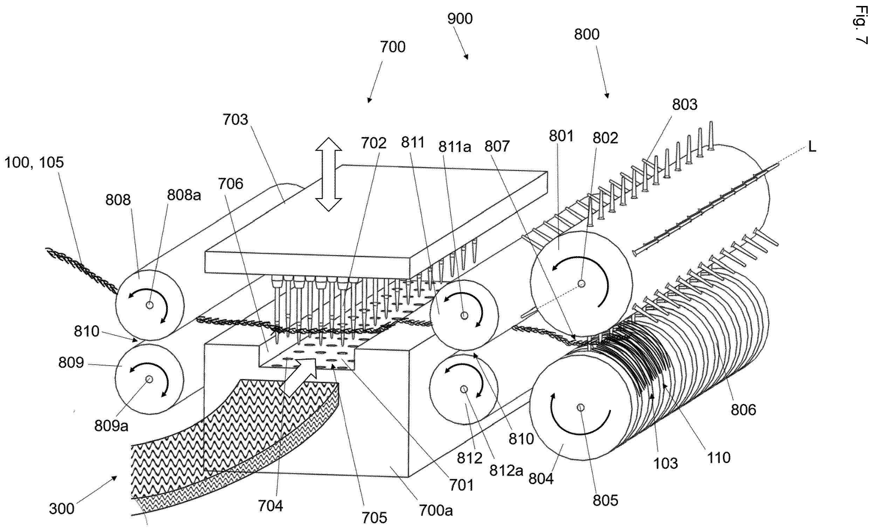

Fig. 7 shows a system 900 comprising a felting device 700 for connecting a medical implant 100 to a medical textile 300 from a felt material and a separation device 800 for generating a first connecting section 110 of the medical implant 100.

The separation device 800 comprises a first roller 801 rotatably mounted on a first shaft 802 extending along a longitudinal axis L and a second roller 804 rotatably mounted on a second shaft 805 which is parallel to the longitudinal axis L. The first roller 801 and the second roller 804 are separated by a gap 807 for receiving the medical implant 100. The first roller 801 comprises spikes 803 extending outward in a radial direction in respect of the longitudinal axis L, and the second roller 804 comprises corresponding grooves 806 extending around the circumference of the second roller 804 in a circumferential direction in respect of the second shaft 805. In the example shown in Fig. 7, the spikes 803 are grouped into six rows forming rotating combs, wherein each row is arranged in a direction parallel to the longitudinal axis L, and the six rows are spaced at equal distances around the circumference of the first roller 801.

The first roller 801 and the second roller 804 are aligned such that the spikes 803 protrude into and move through the grooves 806 when the first roller 801 is rotated around the first shaft 802. In this manner, the spikes 803 engage a medical implant 100 arranged in the gap 807 between the first roller 81 and the second roller 804 and disengage and separate the braided or twisted fibers 101 of the medical implant 100 to generate the first connecting section 110. Of course, the second connecting section 120 can be generated in a similar manner by providing the opposite end of the medical implant 100 in the gap 807 between the first roller 801 and the second roller 804. The first shaft 802 or the second shaft 805 or both the first shaft 802 and the second shaft 805 may be driven, e.g. by a motor.

The felting device 700 comprises a cube shaped bottom part 700a and a top part 703 configured to be arranged on the bottom part 700a. The bottom part 700a forms a slot 705 for receiving a medical textile 300 delimited by a surface 701 and side walls 706 on two opposite sides of the slot 705. The slot 705 is open towards the remaining two sides delimiting the surface 701.

The top part 703 comprises a plurality of felting needles 702 each comprising at least one barb, particularly each comprising a plurality of barbs, wherein the needles 702 are facing with their tips towards the surface 701 of the bottom part 700a when the top part 703 is arranged on the bottom part 700a. The surface 701 further comprises an array of holes 704 aligned with the needles 702 of the top part 703, such that each needles 702 is at least partially inserted into a corresponding hole 704 when the top part is placed on the bottom part 700a or moved towards the bottom part 700a.

To connect a medical implant 100 to a medical textile 300 from a felt material, the medical textile 300 is inserted into the slot of the bottom part 700a, the first connecting section 110 or the second connecting section 120 of the medical implant 100 is arranged on the medical textile 300, and the top part 703 is positioned on the bottom part 700a. Subsequently, the top part 703 with the attached needles 702 is moved up and down periodically through the connecting section 110, 120 and the medical textile 300, such that fibers 101 of the connecting section 110, 120 are moved into the felt material of the medical textile 300 to tightly connect the medical implant 100 to the medical textile 300.

Fig. 7 further displays a conveyor mechanism comprising a third roller 808, a fourth roller 809, a fifth roller 811 and a sixth roller 812, wherein the third roller 808 and the fourth roller 809, and the fifth roller 811 and the sixth roller 812 are arranged in pairs on either side of the felting device 700, wherein the fifth roller 808 and the sixth roller 809 are arranged between the felting device 700 and the separation device 800. The third roller 808 is rotatably mounted on a third shaft 808a, the fourth roller 809 is rotatably mounted on a fourth shaft 809a arranged parallel to the third shaft 808a, the fifth roller 811 is rotatably mounted on a fifth shaft 811a, and the sixth roller 812 is rotatably mounted on a sixth shaft 812a arranged parallel to the fifth shaft 811a, such that respective gaps 810 are formed between the third roller 808 and the fourth roller 809 and between the fifth roller 811 and the sixth roller 812. The third shaft 808a and/or the fourth shaft 809a and the fifth shaft 811a and/or the sixth shaft 812a may be driven, e.g. by a motor. However, it is also conceivable that at least one of the third roller 808 and the fourth roller 809 and the fifth roller 811 and the sixth roller 812 is passive, i.e. the respective shaft 808a, 809a, 811a, 812a is not driven by a motor.

In a preferred method, the conveyor mechanism may be used as follows: a first end 103 of a medical implant 100 is inserted in the gap 810 between the third roller 808 and the fourth roller 809, and then pulled towards the fifth roller 811 and the sixth roller 812 and inserted in the gap 810 between the fifth roller 811 and the sixth roller 812 (either manually or by rotating the third roller 808 and/or the fourth roller 809). By rotating the third roller 808 counterclockwise, the fourth roller 809 clockwise, the fifth roller 811 counterclockwise and the sixth roller 812 clockwise, the first end 103 is then moved towards the gap 807 between the first roller 801 and the second roller 804 of the separation device 800. By separating the fibers 101 of the medical implant 100 on the first end 103 by means of the spikes 803 on the first roller 801, the first connecting section 110 is generated. Subsequently, a felt medical textile 300 is arranged on the surface 701 in the slot 705 of the felting device 700, and the third roller 808, the fourth roller 809, the fifth roller 811 and/or the sixth roller 812 are rotated in the opposite direction to move the first end 103 with the first connecting section 110 onto the medical textile 103 in the slot 705. The top part 703 is then closed, and the medical implant 100 is connected to the medical textile 300 by repeatedly advancing the needles 702.

**List of reference signs**

| | |
|---|---|
| Medical implant | 100 |
| Fiber | 101 |
| Barb | 102 |
| First end | 103 |
| Second end | 104 |
| Main section | 105 |
| Fiber end | 106 |
| First connecting section | 110 |
| Upper portion | 110a |
| Lower portion | 110b |
| Second connecting section | 120 |
| Upper portion | 120a |
| Lower portion | 120b |
| First medical implant | 130 |
| Second medical implant | 140 |
| Felting needle | 200 |
| Medical textile | 300 |
| First medical textile | 301 |
| Second medical textile | 302 |
| Fiber | 303 |
| Opening | 304 |
| Soft biological tissue | 500 |
| Tendon | 501 |
| Humerus bone | 502 |
| Bone tunnel | 502a |
| Medical device | 600 |
| Anchoring element | 610 |
| Felting device | 700 |
| Bottom part | 700a |
| Surface | 701 |
| Needle | 702 |
| Top part | 703 |
| Hole | 704 |
| Slot | 705 |
| Side wall | 706 |
| Separation device | 800 |
| First roller | 801 |
| First shaft | 802 |
| Spike | 803 |
| Second roller | 804 |
| Second shaft | 805 |
| Groove | 806 |
| Gap | 807 |
| Third roller | 808 |
| Third shaft | 808a |
| Fourth roller | 809 |
| Fourth shaft | 809a |
| Gap | 810 |
| Fifth roller | 811 |
| Fifth shaft | 811a |
| Sixth roller | 812 |
| Sixth shaft | 812a |
| System | 900 |
| Longitudinal axis | L |

## Claims

1. A medical implant (100) extending between a first end (103) and a second end (104), wherein the medical implant (100) comprises or consists of a plurality of fibers (101), **characterized in that**
the medical implant (100) comprises a main section (105) and a first connecting section (110) configured to be connected to soft biological tissue (500) or to a medical textile (300), particularly comprising or consisting of a felt material, wherein the first connecting section (110) extends towards the first end (103), and wherein the fibers (101) are connected, particularly braided, twisted, knitted or felted, in the main section (105) and separated from each other in the first connecting section (110).

2. The medical implant (100) according to claim 1, **characterized in that** the medical implant (100) comprises a second connecting section (120) configured to be connected to soft biological tissue (500) or to a medical textile (300), particularly comprising or consisting of a felt material, wherein the second connecting section (120) extends towards the second end (104), wherein the fibers (101) are separated from each other in the second connecting section (120).

3. The medical implant (100) according to claim 1 or 2, **characterized in that** each of the separated fibers (101) comprises a fiber end (106) or a fiber loop positioned at the first end (103) or the second end (104) of the medical implant (100).

4. The medical implant (100) according to any one of the preceding claims, **characterized in that** the main section (105) and the first connecting section (110) and/or second connecting section (120) of the medical implant (100) each comprise an area perpendicular to an extension direction of the medical implant (100), wherein the area of the first connecting section (110) and/or second connecting section (120) is greater than the area of the main section (105).

5. The medical implant (100) according to any one of the preceding claims, **characterized in that**
- the first connecting section (110) or the second connecting section (120) forms a fan-like structure extending in a plane parallel to an extension direction of the medical implant (100), or
- the first connecting section (110) or the second connecting section (120) forms a tubular structure, particularly comprising a circular or oval-shaped cross-sectional contour perpendicular to an extension direction of the medical implant (100), wherein particularly a width of the cross-sectional contour increases, particularly monotonously, towards the first end (103) of the medical implant (100).

6. The medical implant (100) according to any one of the preceding claims, **characterized in that** the fibers (101) each comprise a plurality of barbs (102).

7. The medical implant (100) according to any one of the preceding claims, **characterized in that** the fibers (101) comprise or consist of a biocompatible polymer, particularly polyethylene, polytetrafluorethylene, polyethylene terephthalate, poly(glycolide), poly(lactic-co-glycolic acid), poly (L-lactic acid), polycaprolactone, 1,2-propanediol, or 1,3-propanediol.

8. A medical device (600) comprising at least one medical implant (100) according to any one of the claims 1 to 7, **characterized in that** the medical device (600) further comprises a medical textile (300), particularly comprising or consisting of a felt material, wherein the separated fibers (101) of the first connecting section (110) and/or the second connecting section (120) of the medical implant (100) extend into or through the medical textile (300) to connect the medical implant (100) to the medical textile (300).

9. The medical device (600) according to claim 8, **characterized in that** the medical textile (300) extends along a plane, wherein particularly the medical textile (300) comprises a flat shape.

10. The medical device (600) according to claim 9, **characterized in that**
- the medical device (600) comprises a first medical implant (130) according to any one of the claims 1 to 7, wherein the first connecting section (110) and/or the second connecting section (120) of the first medical implant (130) forms a fan-like structure extending in a plane parallel to an extension direction of the first medical implant (100), and wherein the first medical implant (130) is connected to the medical textile (300), such that the first medical implant (130) extends from the medical textile (300) parallel to the plane of the medical textile (300), and/or
- the medical device comprises a second medical implant (140) according to any one of the claims 1 to 7, wherein the first connecting section (110) or the second connecting section (120) of the second medical implant (140) forms a tubular structure, particularly comprising a circular or oval-shaped cross-sectional contour perpendicular to an extension direction of the second medical implant (140), wherein particularly a width of the cross-sectional contour increases, particularly monotonously, towards the first end (103) of the second medical implant (140) wherein the second medical implant (140) is connected to the medical textile (300), such that the second medical implant (140) extends from the medical textile (300) perpendicular to the plane of the medical textile (300).

11. The medical device (600) according to claim 8, **characterized in that** the medical textile (300) comprises a tubular shape, wherein the medical textile (300) comprises an opening (304) configured for insertion of a soft biological tissue (500) into the medical textile (300), wherein particularly the first connecting section (110) or the second connecting section (120) of the medical implant (100) forms a tubular structure, wherein the tubular structure is arranged in the medical textile (300) contacting the inner circumference of the medical textile (300) or wherein the tubular structure is arranged at least partially around the outer circumference of the medical textile (300), wherein the separated fibers (101) of the tubular structure extend into or through the medical textile (300) to connect the medical implant (100) to the medical textile (300).

12. The medical device (600) according to any one of the claims 8 to 11, **characterized in that** the medical device (600) further comprises at least one anchoring element (610) configured to be inserted into a bone, wherein particularly the anchoring element (610) is connected to the medical implant (100).

13. A separation device (800) for producing a medical implant (100) according to any one of the claims 1 to 7, comprising a first roller (801) rotatably mounted on a first shaft (802) extending along a longitudinal axis (L) and a second roller (804), particularly rotatably mounted on a second shaft (805) parallel to the first shaft (802), wherein the first roller (801) comprises a plurality of spikes (803) extending radially with respect to the longitudinal axis (L), and wherein the second roller (804) comprises a plurality of grooves (806) extending in a circumferential direction around the second roller (804), wherein each groove (806) is aligned with a corresponding spike (803) of the first roller (801), such that the spikes (803) of the first roller (801) are able to insert into and move through the respective grooves (806) when the first roller (801) is rotated around the first shaft (802), and wherein the first roller (801) and the second roller (804) are separated by a gap (807) configured to receive a medical implant (100) comprising a plurality of braided or twisted fibers (101), and wherein the device (800) is configured to separate the fibers (101) of the medical implant (100) by means of the spikes (803) when the first roller (801) is rotated around the first shaft (802), thereby generating the first connecting section (110) or the second connecting section (120) of the medical implant (100).

14. A felting device (700) for producing a medical device according to any one of the claims 8 to 12, wherein the device (700) comprises a surface (701) for arranging a medical textile (300) and at least one needle (702) comprising at least one barb, wherein the device (700) is configured to repeatedly advance the needle (702) through the medical textile (300) arranged on the surface (701) and through the first connecting section (110) or the second connecting section (120) of a medical implant (100) according to any one of the claims 1 to 7 which is arranged on the medical textile (300), such that the separated fibers (101) of the first connecting section (110) or the second connecting section (120) of the medical implant (100) are advanced into or through the medical textile (300) to connect the medical implant (100) to the medical textile (300).

15. A method for biological soft tissue (500) repair, wherein the method comprises providing a medical implant (100) according to any one of the claims 1 to 7 or a medical device (600) according to any one of the claims 8 to 12, and wherein the first connecting section (110) or the second connecting section (120) of the medical implant (100) or the medical textile (300) of the medical device (600) is arranged on a soft biological tissue (500), and wherein a needle (200) comprising at least one barb is repeatedly advanced through the first connecting section (110) or the second connecting section (120) of the medical implant (100) and the soft biological tissue (500) or through the medical textile (300) and the soft biological tissue (500) to connect the medical implant (100) or the medical device (600) to the soft biological tissue (500), wherein particularly the method is performed outside of a human or animal body.
